# EUROPEAN PATENT APPLICATION

(11) **EP 1 522 597 A1**
(43) Date of publication of application: **13.04.2005**
(21) Application number: 04024074.9
(22) Date of filing: 08.10.2004
(51) Int. Cl.: C12Q 1/68

(54) **Nucleic acid amplification method**

(30) Priority: 10.10.2003 JP 2003351988
(71) Applicant: Aisin Seiki Kabushiki Kaisha, Kariya-shi, Aichi-ken 448-8650 (JP)
(72) Inventor: Shigemori, Yasushi, c/o Aisin Cosmos R&D Co.,Ltd., Kariya, Aichi, 448-8650 (JP)
(74) Representative: Leson, Thomas Johannes Alois, Dipl.-Ing.

(57) **Abstract**

A DNA amplification method of amplifying DNA by PCR comprises a process of preparing a mixed solution for PCR reaction by mixing a template DNA, a primer DNA, a RecA protein derived from Thermus thermophilus (T. th. RecA protein) and a phage T4 gene 32 protein, and a process of amplifying DNA by subjecting the prepared mixed solution for PCR reaction to PCR reaction.

[Sequence List]
<110> AISIN SEIKI KABUSHIKI KAISHA
<120> A nucleic acid amplification method and a reagent kit
for amplifying nucleic acids
<130> AK030383
<160> 2
<210> 1
<211> 20
<212> DNA
<213> Artificial Sequence
<220>
<223> Synthesis with reference to Mouse DNA sequence from
clone RP23-253K17 on chromosome 2, complete sequence
(ACCESSION AL929018)
<400> 1
gtgggttttc ctctgtctcc 20
<210> 2
<211> 22
<212> DNA
<213> Artificial Sequence
<220>
<223> Synthesis with reference to Mouse DNA sequence from
clone RP23-459P8 on chromosome 11, complete sequence
(ACCESSION AL669902)
<400> 2

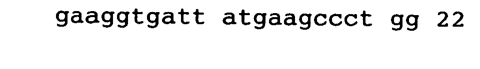

## Description

### FIELD OF THE INVENTION

The present invention relates to a nucleic acid amplification method and a reagent kit for amplifying nucleic acids, and more specifically, a nucleic acid amplification method for amplifying certain nucleic acids by PCR and a reagent kit for amplifying certain nucleic acids by PCR.

### BACKGROUND

A nucleic acid amplification method by PCR (polymerase chain reaction) is conventionally known. It involves admixing a template DNA, a primer DNA, a DNA polymerase, etc. in a reaction solution, and specifically amplifying a region of the template DNA interposed between two kinds (one pair) of the primer DNA, to obtain certain nucleic acids. PCR is a remarkable technique to amplify certain nucleic acids to be targeted more than a hundred thousand times in a short time. However, it is difficult to optimize this reaction, and a technique is needed for specifically amplifying only the desired nucleic acids while suppressing amplification of non-specific nucleic acids.

In view of such problems, a variety of improved methods have been proposed. For example, Non-Patent Document 1 discloses a gist that a single-stranded DNA-binding protein of Escherichia coli is added to a reaction solution to specifically amplify desired nucleic acids. Patent Document 1 also discloses a PCR method using a RecA protein of E. coli, etc. Patent Documents 2 to 5 disclose PCR methods using a phage T4 gene 32 protein.
[Non-Patent Document 1] Escherichia coli single-stranded DNA-binding protein, a molecular tool for improved sequence quality in pyrosequencing. Electrophoresis, 2002 Sep; 23(19): 3289-99
[Patent Document 1] Japanese Patent No. 3010738
[Patent Document 2] JP-A-2003-144169
[Patent Document 3] JP-A-2000-4878
[Patent Document 4] JP-A-2000-342287
[Patent Document 5] JP-A-1993-336971

### SUMMARY OF THE INVENTION

However, even with a variety of the improved conventional methods, it may occur that desired nucleic acids (specific PCR products) are not amplified sufficiently, or the by-products (the non-specific PCR products) are amplified in a significant amount together with the desired nucleic acids (specific PCR products).

In light of such circumstances, an object of the present invention is to provide a nucleic acid amplification method for amplifying desired nucleic acids while suppressing amplification of by-products in the PCR reaction.

Means for solving such problems is a nucleic acid amplification method for amplifying nucleic acids by PCR, which is characterized by admixing in a reaction solution, a first protein which comprises at least any one of a RecA protein derived from Thermus thermophilus and a modified protein obtained by modification of the RecA protein and having a function similar to that of the RecA protein, and a second protein which comprises at least any one of a phage T4 gene 32 protein and a modified phage T4 gene 32 protein obtained by modification of the phage T4 gene 32 protein and having a function similar to that of the phage T4 gene 32 protein; and carrying out PCR.

According to the present invention, a first protein such as RecA protein derived from Thermus thermophilus and a second protein such as phage T4 gene 32 protein are admixed in a reaction solution, and PCR is carried out to amplify desired nucleic acids.

By carrying out PCR as such, the amplification efficiency for the desired nucleic acids (appropriately and specific PCR products) can be improved. On the other hand, amplification of by-products (non-specific PCR products) can be suppressed low. In other words, the first protein such as RecA protein of Thermus thermophilus and the second protein such as phage T4 gene 32 protein act on the template DNA or the primer DNA to promote the binding of the primer DNA to a certain sequence of the template DNA only, thereby promoting amplification of the specific PCR products while suppressing amplification of the non-specific PCR products.

As described in the section of the background art, it is known that the RecA protein of E. coli is added in the PCR reaction. But it is not known that the first protein such as RecA protein derived from Thermus thermophilus is added. In addition, the first protein such as RecA protein derived from Thermus thermophilus has remarkably excellent effects, compared to the RecA protein of E. coli, which is unexpected by the person skilled in the art. In other words, even if the first protein such as RecA protein derived from Thermus thermophilus is added alone without adding the second protein such as phage T4 gene 32 protein, amplification efficiency of the specific PCR products is improved while amplification of the non-specific PCR products is suppressed low, which is unexpected by the person skilled in the art.

As described in the section of the background art, it is a known technique that a phage T4 gene 32 protein is added in the PCR reaction. But, it is not a known technique that the phage T4 gene 32 protein is combined with the first protein such as RecA protein of Thermus thermophilus. Moreover, if the second protein such as phage T4 gene 32 protein is combined with the first protein such as RecA protein of Thermus thermophilus, it has remarkably excellent effects, compared to the case where the phage T4 gene 32 protein is added alone, which is unexpected by the person skilled in the art. In addition, if the second protein such as phage T4 gene 32 protein is combined with the first protein such as RecA protein of Thermus thermophilus, it has remarkably excellent effects even compared with the case where the first protein such as RecA protein of Thermus thermophilus is added alone, which is unexpected to the person skilled in the art.

Herein, the above-mentioned first protein is not limited if it comprises at least any one of the RecA protein of Thermus thermophilus (herein, also referred to as T. th. RecA protein) and the modified RecA protein obtained by modification of the T. th. RecA protein and having a function similar to that of the T. th. RecA protein (modified T. th. RecA protein). The modified T. th. RecA protein includes, for example, a gene product made by inducing site-specific mutation, etc., from a gene which encodes the T. th. RecA protein, comprising an amino acid sequence with deletion, substitution or addition of one or more amino acids, and further has a function similar to that of the T. th. RecA protein. In addition, it may be a protein fragment of the T. th. RecA protein which has a function similar to that of the T. th. RecA protein (a T. th. RecA fragment) and the like.

The first protein is preferably mixed in a range of 0.1 µg to 100 µg per 1 µg of the primer DNA, and more preferably 1 µg to 10 µg per 1 µg of the primer DNA. If PCR is carried out with the first protein in such a range, the desired nucleic acids can be amplified more efficiently and specifically.

The above-mentioned second protein is not limited if it comprises at least any one of the phage T4 gene 32 protein and the modified phage T4 gene 32 protein obtained by modification of the phage T4 gene 32 protein and having a function similar to that of the phage T4 gene 32 protein. The modified phage T4 gene 32 protein includes, for example, a gene product made by inducing site-specific mutation, etc., from the phage T4 gene, comprising an amino acid sequence with deletion, substitution or addition of one or more amino acids, and further having a function similar to that of the phage T4 gene 32 protein. In addition, it may be a protein fragment of the phage T4 gene 32 protein which having a function similar to that of the phage T4 gene 32 protein (a phage T4 gene 32 fragment) and the like.

The second protein is preferably mixed in a range of 0.1 µg to 100 µg per 1 µg of the primer DNA, and more preferably 1 µg to 10 µg per 1 µg of the primer DNA. If PCR is carried out with the second protein in such a range, the desired nucleic acids can be amplified more efficiently and specifically.

Various reagents and the like used in the PCR reaction will be explained hereinbelow.

The template DNA is not particularly limited. In other words, any template DNA comprising any base sequence may be used, and the chain length is not limited by any upper limit. Accordingly, for example, even a ginat DNA having a full length of 3000 Mbp of the human genome may be used. Needless to say, the origin thereof is not limited. Accordingly, it includes a DNA derived from a virus or a microorganism, or an animal or a plant, or a modified DNA thereof; a plasmid, etc. contained in microorganisms, etc., or a chimera DNA formed by insertion of a heterogeneous DNA fragment into the plasmid, etc. contained in microorganisms, etc.; or an artificially synthesized oligonucleotide, etc. In addition, the template DNA may be a double-stranded DNA or a single-stranded DNA. Further, a cDNA obtained by the reverse transcription of an RNA may be also used as a template DNA.

The primer DNA is not particularly limited if it is substantially complementary to a significant number of the bases located at both ends of the sequences to be amplified (the region) in the template DNA, and also the origin thereof is not limited. The extent of the substantial complementarity is preferably a mismatch of three bases or less, more preferably two bases or less, further preferably one base or less for the template DNA. In particular, 100% complementarity is preferable. The reason for this is that amplification of desired nucleic acids becomes difficult with low complementarity of the primer DNA, since, as described above, the specificity of the PCR reaction is improved by the presence of the first protein such as T. th. RecA protein and the second protein such as phage T4 gene 32 protein.

Further, the primer DNA is preferably mixed in a range of 0.01 µM to 10 µM, and more preferably 0.1 µM to 1 µM for each primer DNA. If PCR is carried out with the primer DNA in such a range, the desired nucleic acids can be amplified more efficiently and specifically.

A suitable DNA polymerase is one which is not permanently inactivated by heating for a short time at a high temperature at which the DNA chain is denatured in the PCR, and has activity at high temperatures. The DNA polymerase includes, for example, a DNA polymerase derived from thermophilic bacteria such as Thermococcus litoralis, Bacillus stearothermophilus, Methanothermus fervidus, Thermus aquaticus, T. flavus, T. lacteus, T. rubens, T. ruber and T. thermophilus, a DNA polymerase derived from thermophilic Archaea such as Desulfurococcus mobilis, Methanobacterium thermoautotrophilcum, Sulfolobus solfataricus, S. acidocaldarius and Thermoplasma acidophilum. Among these, a DNA polymerase derived from Thermus aquaticus (a Taq DNA polymerase), a DNA polymerase derived from Thermus thermophilus (a T. th. DNA polymerase), and a DNA polymerase derived from Thermococcus litoralis are preferred, in view of easy availability, etc.

In addition, for example, if a Taq DNA polymerase is used, the DNA polymerase is preferably mixed in a range of 0.05 unit to 50 units per 100 µl*,* and more preferably 0.5 unit to 5 units per 100 µl. If PCR is carried out with the DNA polymerase in such a range, the desired nucleic acids can be amplified more efficiently and specifically.

Further, an antibody which is specific to the above-mentioned DNA polymerase may be mixed in the PCR reaction solution. This is to inhibit the activity of the above-mentioned DNA polymerase before nucleic acid amplification by the DNA polymerase, i.e., to inhibit the production of by-products such as a primer-dimer by an enzymatic reaction at room temperature, or to protect primers from decomposition. Such antibody includes a monoclonal antibody, a polyclonal antibody, an antibody produced by a recombination method, and an antibody fragment produced by a chemical or recombination method (for example, a Fab fragment). Among them, it is particularly preferable to use a monoclonal antibody. For example, if a known monoclonal antibody for the Taq DNA polymerase is used, the enzymatic activity of the Taq DNA polymerase at a temperature of about 20°C to about 40°C can be inhibited, and also can be inactivated by the high temperature of the thermal PCR cycle.

In addition, an antibody which is specific to the first protein such as T. th. RecA protein may be also mixed in the PCR reaction solution.

PCR is generally carried out in the presence of four kinds of dNTPs, i.e., dATP, dCTP, dGTP and dTTP.

In addition, PCR is generally carried out in a reaction solution containing a suitable buffer to amplify nucleic acids efficiently. The buffer solution can be suitably varied to obtain optimal reaction conditions by the first protein, the second protein, the DNA polymerase, etc. used in the PCR reaction. For example, potassium chloride or magnesium chloride can be added to a TRIS buffer solution of which pH is suitably adjusted. The buffer agent includes TRICINE, BIS-TRICINE, HEPES, MOPS, TES, TAPS, PIPES and CAPS. The salt which may be suitably used includes potassium acetate, potassium sulfate, ammonium sulfate, ammonium chloride, ammonium acetate, magnesium acetate, magnesium sulfate, manganese chloride, manganese acetate, manganese sulfate, sodium chloride, sodium acetate, lithium chloride and lithium acetate.

In addition, various additives may be added to the PCR reaction solution. For example, 5% to 10% of DMSO and 1% to 2% of betaine may be added to minimize the problem that amplification of the desired product becomes poor due to a secondary structure of the template DNA. The first protein such as T. th. RecA protein has resistance to denaturing agents and thus can be used in the present invention while the RecA protein derived from E. coli has no resistance to denaturing agents. The additive which may be suitably used includes glycerol, formaldehyde, tetramethylammonium chloride, dimethylsulfoxide, polyethyleneglycol (PEG), Tween20, Nonidet-P40 (NP40), ectoine, Triton-X100, polyols, etc.

ATP-γS may be also added to the PCR reaction solution to increase the possibility of more specific amplification of desired nucleic acids. The addition of ATP is also assumed to increase the specificity of PCR in the similar manner as in the addition of ATP-γS. However, ATP is decomposed to ADP by the first protein such as RecA protein, and the ADP inhibits the binding of the first protein such as T. th. RecA protein to the primer DNA. Therefore, addition of ATP may not always improve the specificity of PCR. Accordingly, ATP-γS which is not decomposed to ADP is preferably added to the reaction solution.

In addition, the concentration of ATP-γS is varied suitably depending on the purpose, but usually 0.01 mM to 10 mM, and preferably 0.1 mM to 1 mM.

Another means for solving such problems is a reagent kit for amplifying nucleic acids by PCR, which is characterized by comprising a DNA polymerase, four kinds of dNTPs, a buffer solution, a first protein comprising at least any one of a RecA protein derived from Thermus thermophilus and a modified RecA protein obtained by modification of the RecA protein and having a function similar to that of the RecA protein, and a second protein comprising at least any one of a phage T4 gene 32 protein and a modified phage T4 gene 32 protein obtained by modification of the phage T4 gene 32 protein and having a function similar to that of the phage T4 gene 32 protein.

The reagent kit for amplifying nucleic acids of the present invention comprises a DNA polymerase, four kinds of dNTPs, a buffer solution, a first protein such as T. th. RecA protein, and a second protein such as phage T4 gene 32 protein.

PCR can be easily carried out using such kit, just by adding a template DNA and a primer DNA prepared depending on the purpose to the reaction solution, in addition to the DNA polymerase, the four kinds of dNTPs, the buffer solution, the first protein such as T. th. RecA protein, and the second protein such as phage T4 gene 32 protein. Further, the first protein such as RecA protein or the second protein such as phage T4 gene 32 protein acts on the template DNA or the primer DNA to promote the binding of the primer DNA to certain sequences of the template DNA only, thereby amplification of the specific PCR products can be promoted while amplification of the non-specific PCR products is suppressed. Accordingly, the desired nucleic acids can be amplified more specifically using the kit.

The reagent kit for amplifying nucleic acids of the present invention may comprise a DNA polymerase, four kinds of dNTPs, a buffer solution, a first protein such as T. th. RecA protein, and a second protein such as phage T4 gene 32 protein. Accordingly, such components may be contained in separate vessels, or two or more components of them may be mixed in advance.

The DNA polymerase, the four kinds of dNTPs, the buffer solution, the first protein such as T. th. RecA protein, and the second protein such as phage T4 gene 32 protein in the present invention are as described above.

### BRIEF DESCRIPTION OF THE DRAWINGS

The foregoing and additional features and characteristics of the present invention will become more apparent from the following detailed description considered with reference to the accompanying drawings, wherein:
Fig. 1 is a drawing showing the relation between the template DNA and the primer DNA with reference to Examples.
Fig. 2 is a photograph instead of a drawing showing the results of electrophoresis for the PCR reaction products with reference to Examples.

### DESCRIPTION OF THE PREFERRED EMBODIMENTS

Examples of the present invention will be further illustrated below with reference to Drawings.

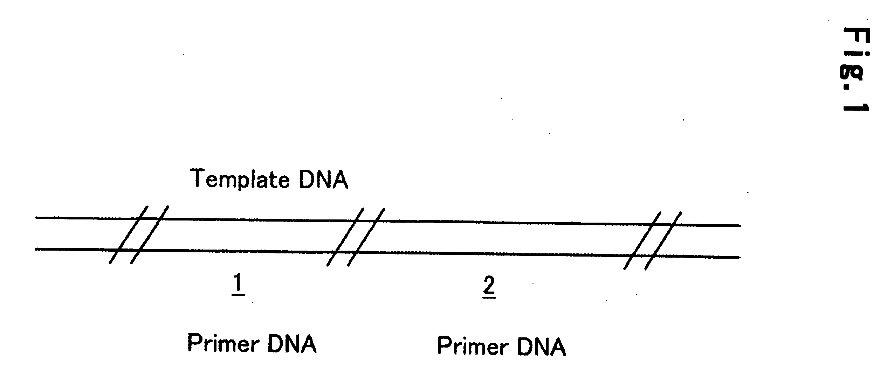

A mouse genome DNA (Promega) was prepared as a template DNA as shown in Fig. 1. A set (2 kinds) of oligonucleotides (Oligonucleotide 1 and Oligonucleotide 2) were prepared as a primer DNA. Primer DNA 1 (Oligonucleotide 1) was designed with reference to the mouse DNA sequence from clone RP23-253K17 on chromosome 2, complete sequence (ACCESSION AL929018). Primer DNA 2 (Oligonucleotide 2) was designed with reference to the mouse DNA sequence from clone RP23-459P8 on chromosome 11, complete sequence (ACCESSION AL669902). The ACCESSION number indicates an access number of Gene Bank. Each primer DNA consists of a base sequence which is 100 % complementary to the template DNA. Each primer DNA may be synthesized by a known method on the basis of the base sequence of the template DNA.

Oligonucleotide 1:

5'-gtgggttttcctctgtctcc-3'

Oligonucleotide 2:

5'-gaaggtgattatgaagccctgg-3'

In addition, a RecA protein of Thermus thermophilus (T. th. RecA protein) was prepared as the first protein such as T. th. RecA protein, and a phage T4 gene 32 protein was prepared as the second protein such as phage T4 gene 32 protein. Further, Expand long template enzyme (Roche) was prepared as a DNA polymerase. In addition, four kinds of dNTPs, i.e., dATP, dCTP, dGTP and dTTP were prepared, and Expand long template buffer (Roche) was prepared as a buffer solution.

Further, it is convenient to prepare a DNA polymerase, four kinds of dNTPs, a buffer solution, a first protein such as T. th. RecA protein, and a second protein such as phage T4 gene 32 protein as a reagent kit for amplifying nucleic acids. PCR can be easily carried out by using such a kit, by preparing a reaction solution wherein the DNA polymerase, the four kinds of dNTPs, the buffer solution, the first protein such as T. th. RecA protein, and the second protein such as phage T4 gene 32 protein are mixed, and by just adding the template DNA and the primer DNA prepared depending on the purpose to the reaction solution.

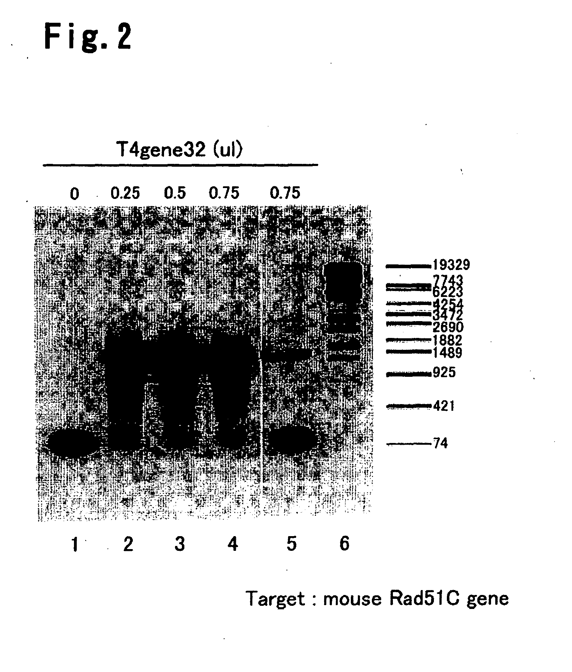

Then, nucleic acids were amplified by the PCR reaction. Specifically, in a 50 µl PCR reaction solution, 0.5 µM (the final concentration) of Oligonucleotide 1 (Primer DNA 1), 0.5 µM (the final concentration) of Oligonucleotide 2 (Primer DNA 2), 50 ng of the mouse genome DNA, 3.75 units of Expand long template enzyme, 0.2 mM of a dNTP mixed solution, and 0.5 µg of the T. th. RecA protein were mixed with 1 X Expand long template buffer. Then, the PCR reaction was carried out with 1 cycle (94°C, 30 seconds), 30 cycles (94°C, 15 seconds; 60°C, 30 seconds; 68°C, 90 seconds) and 1 cycle (68°C, 7 minutes). Then, the reaction solution was subjected to electrophoresis with a 1% agarose gel, and the agarose gel was soaked in an ethidium bromide solution to stain the DNA in the gel, and the stained DNA was recorded by photography. The results are shown in Fig. 2.

In Fig.2, Lane 1 shows the results of the reaction as described above, i.e., the results when the T. th. RecA protein was added without adding the phage T4 gene 32 protein in the PCR reaction solution. The lane number is indicated below in Fig. 2.

Lane 2 shows the results when 0.25 µg of the phage T4 gene 32 protein was added to the above-described PCR reaction solution, and the PCR was carried out.

Lane 3 shows the results when 0.5 µg of the phage T4 gene 32 protein was added to the above-described PCR reaction solution, and the PCR was carried out.

Lane 4 shows the results when 0.75 µg of the phage T4 gene 32 protein was added to the above-described PCR reaction solution, and the PCR was carried out.

Lane 5 shows the results when 0.75 µg of the phage T4 gene 32 protein was added without adding the T. th. RecA protein in the PCR reaction solution, and the PCR was carried out.

Lane 6 shows the results of the electrophoresis of Lambda DNA cleaved by EcoT14I as a DNA size marker.

As clearly shown in the results of Fig. 2, when the T. th. RecA protein was added without adding the phage T4 gene 32 protein in the PCR reaction solution, the desired nucleic acids of about 1.6 kbp (the appropriately specific PCR products) was scarcely detected in Lane 1. When the phage T4 gene 32 protein is added without adding the T. th. RecA protein in the PCR reaction solution, the desired nucleic acids of about 1.6 kbp was detected weakly in Lane 5.

On the other hand, when both the T. th. RecA protein and the phage T4 gene 32 protein were added to the PCR reaction solution, the desired nucleic acids of about 1.6 kbp were detected strongly, and the by-products (the non-specific PCR products) were scarcely detected in Lanes 2 to 4.

From these results, it can be understood that the addition of the T. th. RecA protein alone may not always improve sufficiently the amplification efficiency of the desired nucleic acids. Similarly, it can also be understood that addition of the phage T4 gene 32 protein alone may not always improve sufficiently the amplification efficiency of the desired nucleic acids. On the other hand, if the T. th. RecA protein and the phage T4 gene 32 protein are used in combination, it can be understood that the amplification efficiency of the desired nucleic acids can be improved while suppressing amplification of the by-products.

The present invention was illustrated in the above by Examples which do not limit the present invention, and, needless to say, it can be suitably modified and applied without departing from the spirit or scope of the present invention.

For example, the T. th. RecA protein which was separately extracted and purified was added to the reaction solution, and a PCR reaction was carried out in the above Examples. However, E. coli, etc. which is transformed to express the first protein such as the T. th. RecA protein and then heat-treated, can be also used as a T. th. RecA protein. In other words, this is a method wherein E. coli, etc. is subjected to heat-treatment, thereby leaving T. th. RecA protein, etc. that has high heat-resistance while inactivating other proteins. Especially, when a genome DNA or a plasmid DNA of E. coli is used as a template. DNA, the template DNA and the T. th. RecA protein can be obtained at the same time by heat-treating E. coli, etc. that express the T. th. RecA protein, etc., and thereby the working efficiency for carrying out PCR can be improved.

A DNA amplification method of amplifying DNA by PCR comprises a process of preparing a mixed solution for PCR reaction by mixing a template DNA, a primer DNA, a RecA protein derived from Thermus thermophilus (T. th. RecA protein) and a phage T4 gene 32 protein, and a process of amplifying DNA by subjecting the prepared mixed solution for PCR reaction to PCR reaction.

## Claims

1. A DNA amplification method of amplifying DNA by subjecting a mixed solution of a template DNA, a primer DNA, a DNA polymerase and a buffer solution to PCR reaction,
**characterized in that**
a first protein comprising at least any one of a RecA protein derived from Thermus thermophilus (T. th. RecA protein) and a modified RecA protein obtained by modification of the T. th. RecA protein and having a function similar to that of the T. th. RecA protein, and a second protein comprising at least any one of a phage T4 gene 32 protein and a modified phage T4 gene 32 protein obtained by modification of the phage T4 gene 32 protein and having a function similar to that of the phage T4 gene 32 protein are mixed in the mixed solution.

2. The method according to Claim 1, wherein the first protein is mixed in a range of 0.1 to 100 µg per 1 µg of the primer DNA.

3. The method according to Claim 1, wherein the first protein is mixed in a range of 1 to 10 µg per 1 µg of the primer DNA.

4. The method according to Claims 1 to 3, wherein the second protein is mixed in a range of 0.1 to 100 µg per 1 µg of the primer DNA.

5. The method according to Claims 1 to 3, wherein the second protein is mixed in a range of 1 to 10 µg per 1 µg of the primer DNA.

6. The method according to Claims 1 to 5, wherein the DNA polymerase is one or more selected from the group consisting of DNA polymerase derived from Thermus aquaticus, DNA polymerase derived from Thermus thermophilus and DNA polymerase derived from Thermococcus litoralis.

7. The method according to Claims 1 to 6, wherein ATP-γS is further added to the mixed solution.

8. A reagent kit for DNA amplification to amplify DNA by PCR, **characterized in that**
the reagent kit comprises a DNA polymerase, four kinds of dNTP, a buffer solution, a first protein comprising at least any one of a RecA protein derived from Thermus thermophilus (T. th. RecA protein) and a modified RecA protein obtained by modification of the T. th. RecA protein and having a function similar to that of the T. th. RecA protein, and a second protein comprising at least any one of a phage T4 gene 32 protein (T4gene32 protein) and a modified phage T4 gene 32 protein obtained by modification of the T4gene32 protein and having a function similar to that of the T4gene32 protein.
